# EUROPEAN PATENT SPECIFICATION

(11) **EP 3 142 095 B1**
(45) Date of publication and mention of the grant of the patent: **30.12.2020**
(21) Application number: 16184664.7
(22) Date of filing: 18.08.2016
(51) Int. Cl.: G09B 19/00, G09B 5/02, A63B 22/02, A61B 5/103, A61B 5/00, A63B 69/00, A63B 71/06, A61H 1/02, A61B 5/11, A63B 22/00, G16H 20/30, A63B 24/00, A63B 21/00

(54) **WALKING TRAINING APPARATUS AND WALKING TRAINING METHOD**
LAUFTRAININGSVORRICHTUNG UND LAUFTRAININGSVERFAHREN
APPAREIL D'ENTRAÎNEMENT À LA MARCHE ET SON PROCÉDÉ

(30) Priority: 08.09.2015 JP 2015176967
(43) Date of publication of application: 15.03.2017
(73) Proprietor: Toyota Jidosha Kabushiki Kaisha, Toyota-shi, Aichi-ken, 471-8571 (JP)
(72) Inventor: KONISHI, Kenta, Toyota-shi, Aichi-ken,, 471-8571 (JP); MATSUURA, Junji, Toyota-shi, Aichi-ken,, 471-8571 (JP)
(74) Representative: TBK

(56) References cited:
- DE-A1-102011 106 228
- US-A1- 2009 124 938
- US-A1- 2012 021 873
- US-A1- 2015 196 804
- US-B1- 6 231 527
- Brent Rose: "I Wore a Bionic Leg, And I Never Wanted To Take It Off Again", , 7 October 2013 (2013-10-07), XP055338773, Retrieved from the Internet: URL:http://gizmodo.com/i-wore-a-bionic-leg -and-i-never-wanted-to-take-it-off-7265368 22 [retrieved on 2017-01-25]

## Description

### BACKGROUND OF THE INVENTION

### 1. Field of the Invention

The present invention relates to a walking training apparatus and a walking training method by which a user does a walking training.

### 2. Description of Related Art

A walking training apparatus including display means disposed in front of a person who does a walking training (hereinafter referred to as a "walking trainee"), image pickup means for taking an image of the walking trainee from a viewpoint located above the walking trainee, and display control means for displaying a target landing point of a foot of the walking trainee together with the image of the walking trainee taken by the image pickup means on a display screen of the display means has been known (see, for example, Japanese Unexamined Patent Application Publication JP 2006-204730 A).

Incidentally, a type of walking trainees such as patients having hemiplegia tend to walk in a state where their bodies are inclined either to the left or to the right during walking trainings. However, the walking trainee himself/herself can hardly notice this left/right inclination of his/her body during the walking training. To cope with this, it is conceivable that, for example, an image of the walking trainee taken from a viewpoint located in front of or behind the walking trainee can be displayed on separately-provided display means so that the walking trainee can check the left/right inclination of his/her body. In such a case, however, the walking trainee has a troublesome task of checking the target landing point and the left/right inclination of his/her body in the different display means.

US 2009/0124938 A1 discloses a walking training apparatus in which on a virtual walking route prints of the walking trainee are made visible. These prints of the walking trainee move synchronously with the actual footprints of the walking trainee on a belt. The virtual walking route further includes virtual areas where the walking trainee shall not step on, or shall step on.

US 6 231 527 B1 discloses an apparatus for analyzing abnormal conditions of gait and posture and for translating such analysis into biomechanical correction through the utilization of orthotics alone and in combination with other measures and which includes a walking platform upon which a patient can stride, video cameras directed at the patient including a frontal camera, lateral camera, overhead camera and lower rear camera, mirrors to produce reflected images of the patient from selected of the cameras, and a closed circuit television for simultaneously displaying images of the patient when striding on the walking platform; and such information is coordinated with more traditional diagnostic measures for determining or sensing ground reactive and weight-bearing forces on the feet both when static and during ambulation to produce an accurate prescription through the use of orthotics alone and in combination with other measures.

Brent Rose discloses in "I Wore a Bionic Leg And I Never Wanted To Take It Off Again" discloses another structure of a walking training apparatus with motorized assistance with extension for straightening a knee, for swinging the leg forward between steps, and going up stairs.

US 2012/021873 A1 discloses an arrangement for training the gait, comprising an endless belt guided over at least two rollers and serving as a treadmill, the one surface of which serves as a walking surface, a sensor system for determining a pressure/force distribution on a force measurement plate located underneath the walking surface, which is provided with a plurality of pressure/force sensors on the side facing the endless belt, an analyzing unit connected, on the input side, to the pressure/force sensors, which detects pressure distributions generated by a walking or running subject on the walking surface, a processing unit connected, on the input side, to the analyzing unit, which generates from the pressure distributions values or signals, respectively, that characterize the gait of the subject, optical display means for displaying images in the area of the walking surface, which are connected to image signal generating means and a display process controller for the generation and the position control in terms of time and space of images or image elements, and a synchronization and adaptation stage for synchronizing and adapting the processing of the pressure distribution images with/to the image representation in the area of the walking surface.

DE 10 2011 106228 A1 discloses a method for measuring the exerted forces or moments. The measured forces or moments are evaluated in real time. A gait parameter is visually represented as feedback for the user. The visual representation of gait parameters takes place by an animation, particularly self-explanatory animation. The gait parameter includes a force profile, a temporal symmetry, a force symmetry, a foot lift or weight distribution.

US 2015/0196804 A1 discloses techniques and systems for evaluating exercise performance of a user by utilizing at least one camera-based sensor that detects image data of the user and may show a current image or avatar of the user overlaid onto an image of a reference user that is exercising (e.g., running) at a pace according to the prescribed exercise routine and with correct body form.

### SUMMARY OF THE INVENTION

The present invention has been made in view of the above-described problem and it is the object thereof is to provide a walking training apparatus for allowing a walking trainee to easily check a left/right inclination of his/her body by looking at a center line displayed near the target landing point of his/her foot.

This object is solved by a walking training apparatus having the features of claim 1. A walking training method is stated in claim 8. Further developments are stated in the dependent claims.

With such a walking training apparatus as in claim 1, the walking trainee can easily check the left/right inclination of his/her body by looking at the center line displayed near the target landing point of his/her foot.

The walking training apparatus may further include inclination calculation means for calculating an amount of a left/right inclination and a direction thereof of the body of the walking trainee based on information detected by at least one of a pressure sensor that detects a pressure on a sole of the foot of the walking trainee, an angular speed sensor attached to the walking trainee, second image pickup means for taking an image of the walking trainee from front of or behind the walking trainee, and the first image pickup means, and the display control means may be adapted to control the display means so that a mark indicating the amount of the inclination calculated by the inclination calculation means and the direction of the inclination or a counter-inclination direction adjacent to the center line are displayed on the display screen of the display means.

With such a walking training apparatus, since the walking trainee can visually recognize the amount and direction of the left/right inclination of his/her body just by looking at the mark displayed near the target landing point of the foot and the center line, the walking trainee can check the left/right inclination of his/her body more easily.

The walking training apparatus may further include at least one of sound output means for changing a sound output according to the inclination amount and the inclination direction calculated by the inclination calculation means, vibration means for changing a vibration given to the walking trainee, and optical output means for changing an optical output. With such a walking training apparatus, since the walking trainee can recognize the amount and direction of the left/right inclination of his/her body by the sound, the vibration, the light, and/or the like more intuitively, the walking trainee can check the left/right inclination of his/her body more easily.

The walking training apparatus may further comprise a walking assistance apparatus having the pressure sensor on the sole thereof. With such a walking training apparatus, even a walking trainee having difficulties in walking such as a patient having hemiplegia is able to do a walking training.

The walking training apparatus may further comprise a movable belt on which the walking trainee should land his/her foot, wherein the display control means is adapted to calculate the target landing point of the foot based on the moving speed of the movable belt.

The walking training apparatus may further comprise a switch for switching on and off the center line. With such a walking training apparatus, the walking trainee can do a walking training independently from the own visual recognition of the amount and direction of the left/right inclination of his/her body. Thus, the walking trainee can concentrate only on instructions about the left/right inclination of his/her body given by a physical therapist.

In the walking training apparatus the switch may be further adapted to switch on and off the mark indicating the calculated amount of the inclination. With such a walking training apparatus, the walking trainee can do a walking training independently from the calculated amount of the inclination of his/her body. Thus, the walking trainee can concentrate only on respective instructions given by a physical therapist. To achieve the above-described object, another exemplary aspect of the present invention is a walking training method in which a target landing point is displayed together with an image of a walking trainee on a display screen of display means disposed in front of the walking trainee, the image of the walking trainee being taken by first image pickup means for taking the image of the walking trainee from a viewpoint located above the walking trainee, the target landing point being a point on which the walking trainee should land his/her foot in a next step, in which in addition to the target landing point of the foot, a center line is displayed on the display screen of the display means, the center line indicating a center position in a left/right direction of the walking trainee. According to this aspect, the walking trainee can easily check the left/right inclination of his/her body by looking at the center line displayed near the target landing point of his/her foot.

The present invention has been made in view of the above-described problem and can provide a walking training apparatus and a walking training method for allowing a walking trainee to easily check a left/right inclination of his/her body by looking at a center line displayed near the target landing point of his/her foot.

The above and other objects, features and advantages of the present invention will become more fully understood from the detailed description given hereinbelow and the accompanying drawings which are given by way of illustration only, and thus are not to be considered as limiting the present invention.

### BRIEF DESCRIPTION OF THE DRAWINGS

Fig. 1 is a perspective view showing a schematic configuration of a walking training apparatus according to a first exemplary embodiment of the present invention;
Fig. 2 is a block diagram showing a schematic system configuration of the walking training apparatus according to the first exemplary embodiment of the present invention;
Fig. 3 is a perspective view showing a schematic configuration of a walking assistance apparatus according to the first exemplary embodiment of the present invention;
Fig. 4 shows an example of a target landing point of a foot and a center line displayed in a monitor;
Fig. 5 is a flowchart showing a flow of a walking training method according to the first exemplary embodiment of the present invention;
Fig. 6 shows an example of a target landing point of a foot, a center line, and a mark displayed in a monitor; and
Fig. 7 shows an example of an inclination angle of a center line with respect to a vertical line from a viewpoint located in front of or behind a walking trainee.

### DESCRIPTION OF THE EXEMPLARY EMBODIMENTS

### First exemplary embodiment

A first exemplary embodiment according to the present invention is explained hereinafter with reference to the drawings.

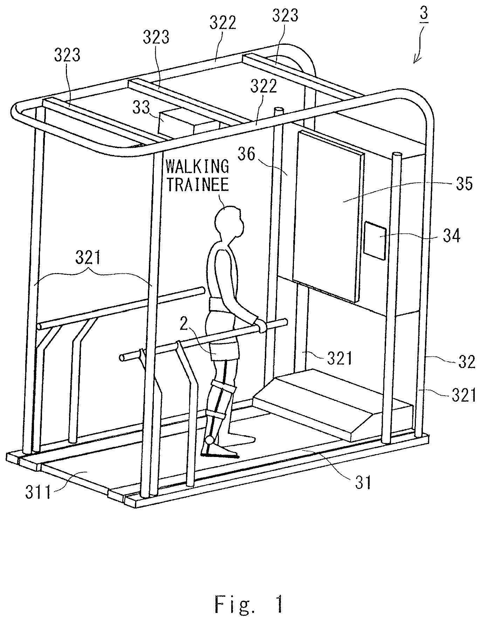

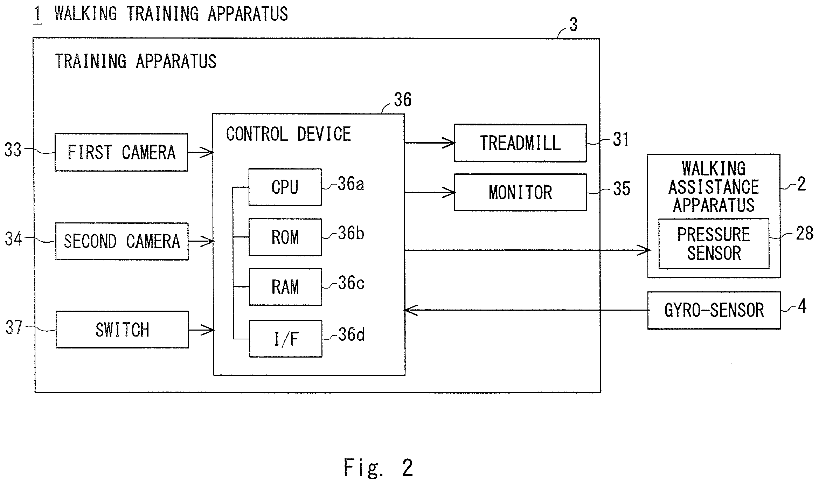

Fig. 1 is a perspective view showing a schematic configuration of a walking training apparatus according to a first exemplary embodiment of the present invention. Fig. 2 is a block diagram showing a schematic system configuration of the walking training apparatus according to the first exemplary embodiment of the present invention. A walking training apparatus 1 according to the first exemplary embodiment is, for example, an apparatus by which a walking trainee such as a patient having hemiplegia caused by a stroke does a walking training. The walking training apparatus 1 includes a walking assistance apparatus 2 attached to a leg of the walking trainee, a training apparatus 3 by which the walking trainee does a walking training, and a gyro-sensor 4 attached to the walking trainee.

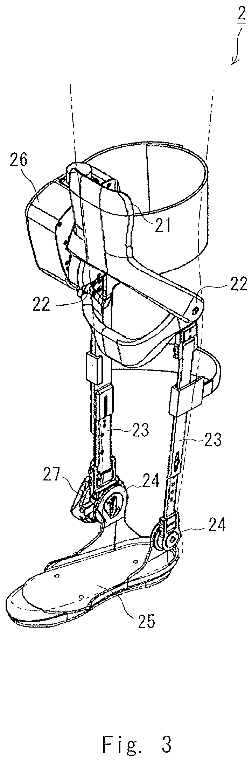

The walking assistance apparatus 2 is, for example, attached to a diseased leg of a walking trainee and assists walking of the walking trainee (Fig. 3). The walking assistance apparatus 2 includes an upper thigh frame 21, a lower thigh frame 23 connected to the upper thigh frame 21 through a knee joint part 22, a sole frame 25 connected to the lower thigh frame 23 through an ankle joint part 24, a motor unit 26 that rotationally drives the knee joint part 22, and an adjustment mechanism 27 that adjusts the movable range of the ankle joint part 24. Note that the above-described configuration of the walking assistance apparatus 2 is merely an example and the configuration of the walking assistance apparatus 2 is not limited to such an example. For example, the walking assistance apparatus 2 may include another motor unit that rotationally drives the ankle joint part 24.

A plurality of pressure sensors 28 is provided in the sole frame 25. The pressure sensors 28 are a specific example of the pressure detection means. Each of the pressure sensors 28 is, for example, a vertical pressure sensor that detects a vertical pressure exerted on the sole of the sole frame 25.

The upper thigh frame 21 is attached to the upper thigh of the leg of the walking trainee and the lower thigh frame 23 is attached to the lower thigh of the leg of the walking trainee. The motor unit 26 rotationally drives the knee joint part 22 according to the walking motion of the walking trainee and thereby assists the walking of the walking trainee. Note that the above-described configuration of the walking assistance apparatus 2 is merely an example and the configuration of the walking assistance apparatus 2 is not limited to such an example. Any walking assistance apparatus capable of being attached to the leg of the walking trainee and assisting walking of the walking trainee can be applied.

The gyro-sensor 4 is a specific example of the angular speed sensor. The gyro-sensor 4 is attached to, for example, the trunk of the walking trainee. The gyro-sensor 4 detects an inclination angular speed of the trunk of the walking trainee. Note that the gyro-sensor 4 may be attached to the head of the walking trainee.

The training apparatus 3 includes a treadmill 31, a frame main body 32, a first camera 33, a second camera 34, a monitor 35, and a control device 36. The treadmill 31 rotates a ring-shaped belt 311. The walking trainee gets on the belt 311 and walks on the belt 311 according to the movement of the belt 311. By doing so, the walking trainee does a walking training.

The frame main body 32 includes two pairs of pillar frames 321 vertically disposed on the treadmill 31, a pair of lengthwise frames 322 extending in the lengthwise direction and connected to respective pillar frames 321, and three crosswise frames 323 extending in the crosswise direction and connected to each of the lengthwise frames 322. Note that the configuration of the above-described frame main body 32 is not limited to this example.

The first camera 33 is a specific example of the first image pickup means. The first camera 33 is disposed, for example, in the crosswise frame 323 of the frame main body 32. The first camera 33 takes an image of the walking trainee walking on the treadmill 31 from a viewpoint located above the walking trainee. The first camera 33 then outputs the image of the walking trainee taken from the upper viewpoint to the control device 36.

The second camera 34 is a specific example of the second image pickup means. The second camera 34 is disposed, for example, in the front part of the frame main body 32. The second camera 34 takes an image of the walking trainee walking on the treadmill 31 from the front of the walking trainee. Alternatively, the second camera 34 may be disposed, for example, in the rear part of the frame main body 32 and takes an image of the walking trainee walking on the treadmill 31 from behind the walking trainee. The second camera 34 then outputs the image of the walking trainee taken from the front viewpoint or the rear viewpoint to the control device 36.

The monitor 35 is a specific example of the display means. The monitor 35 is disposed, for example, in the front part of the frame main body 32 and in front of the walking trainee. The monitor 35 is a liquid-crystal display device, an organic EL display device, or the like. The monitor 35 displays, for example, information such as an image of the walking trainee from the upper viewpoint, the front viewpoint, or the rear viewpoint, a training instruction (such as a target landing point on which the walking trainee should land his/her foot in the next step), a training menu, and training information (such as a walking speed and biometric information). For example, the monitor 35 is constructed as a touch panel and the walking trainee can enter various information items through the monitor 35.

The control device 36 is a specific example of the display control means. The control device 36 controls each of the monitor 35, the driving of the treadmill 31, and the walking assistance apparatus 2. For example, the control device 36 is formed by hardware mainly using a microcomputer including a CPU (Central Processing Unit) 36a that performs arithmetic processing, control processing, and so on, a ROM (Read Only Memory) 38b that stores an arithmetic program, a control program and so on to be executed by the CPU 36a, a RAM (Random Access Memory) 36c that stores various data and so on, and an interface unit (I/F) 36d that externally receives and outputs signals. The CPU 36a, the ROM 36b, the RAM 36c, and the interface unit 36d are connected with each other through a data bus or the like.

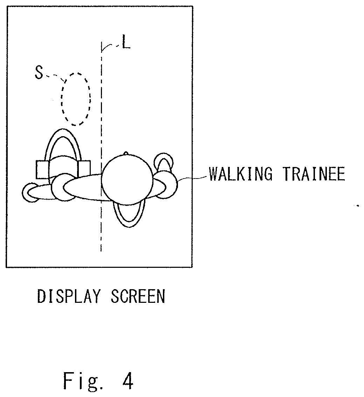

The control device 36 calculates (or determines) a target landing point S on which the walking trainee should land his/her foot in the next step based on the image of the walking trainee from the upper viewpoint output from the first camera 33 (Fig. 4). For example, the control device 36 estimates the length of forward swinging of the leg of the walking trainee based on the moving speed of the belt 311 of the treadmill 31. Then, the control device 36 calculates the target landing point S of the foot of the walking trainee based on the image of the walking trainee from the upper viewpoint output from the first camera 33 and the estimated length of the swinging of the leg. Note that the above-described method for calculating a target landing point S is merely an example and the calculation method is not limited to this example.

The control device 36 displays the target landing point S of the foot of the walking trainee together with the image of the walking trainee from the upper viewpoint taken by the first camera 33 on the display screen of the monitor 35 during the walking training. The walking trainee walks so that his/her own foot lands on the target landing point S displayed in the monitor 35.

Incidentally, for example, a type of walking trainees such as patients having hemiplegia tend to walk in a state where their bodies are inclined either to the left or to the right during walking trainings. However, the walking trainee himself/herself can hardly notice the left/right inclination of his/her body during the walking training.

To cope with this, in the walking training apparatus 1 according to the first exemplary embodiment, the control device 36 displays a center line L indicating the center position in the left/right direction of the walking trainee in addition to the target landing point S of his/her foot on the display screen of the monitor 35 (Fig. 4). Therefore, the walking trainee can easily check the inclination of his/her body in the left/right direction by looking at the center line L displayed near the target landing point S of his/her foot.

For example, in the related art, an image of a walking trainee from a viewpoint located in front of or behind the walking trainee is displayed in a separately-provided monitor, and the walking trainee has a troublesome task of checking the target landing point and the left/right inclination of his/her body in the different monitors. In contrast to this, according to the walking training apparatus 1 in accordance with the first exemplary embodiment, as described above, as the walking trainee looks at the target landing point S of his/her foot on the screen of the monitor 35 located in front of him/her during a walking training, the center line L, which is displayed near the target landing point S, also comes into sight of the walking trainee. Therefore, the walking trainee can check the inclination of his/her body in the left/right direction without paying attention thereto.

The control device 36 calculates a center line L indicating the center position in the left/right direction of the walking trainee based on the upper-viewpoint image of the walking trainee output from the first camera 33. For example, the control device 36 determines the positons of the left and right feet of the walking trainee based on the image of the walking trainee from the upper viewpoint output from the first camera 33 and calculates a line extending from the center of the positons of the left and right feet in the front/rear direction (i.e., in the walking direction) as the center line L. Note that the above-described method for calculating a center line L is merely an example and the calculation method is not limited to this example. For example, the control device 36 may calculate a center line L based on information output from the gyro-sensor 4, the pressure sensors 28 of the walking assistance apparatus 2, or the second camera 34.

Note that the control device 36 may include a switch 37, and when it is switched on, the center line L may disappear from the display screen of the monitor 35 so that only the target landing point S of the foot is displayed on the display screen of the monitor 35. When a walking trainee does a walking training in the presence of a physical therapist, the physical therapist may give instructions about the left/right inclination of the body to the walking trainee. In such a case, the center line L disappears from the display screen of the monitor 35 when the above-described switch 37 is switched on, so that only the target landing point S of the foot is displayed on the display screen of the monitor 35. In this way, the walking trainee can do a walking training while concentrating only on the instructions about the left/right inclination of his/her body given by the physical therapist.

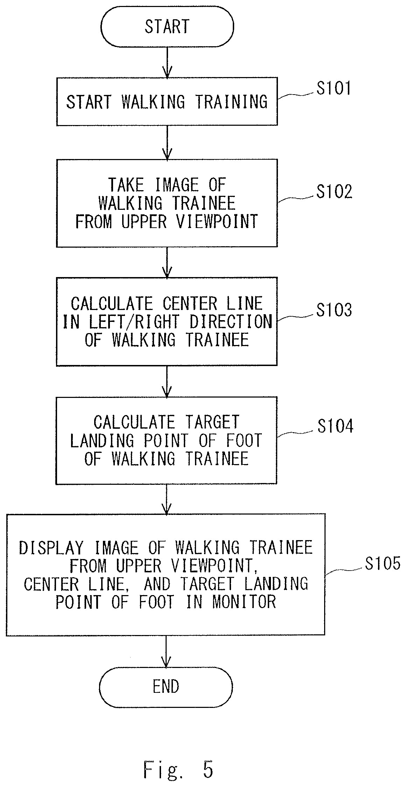

Fig. 5 is a flowchart showing a flow of a walking training method according to the first exemplary embodiment of the present invention.

When a walking training is started, the treadmill 31 is driven and a walking trainee starts walking on the treadmill 31 (step S101).

The first camera 33 takes an image of the walking trainee walking on the treadmill 31 from a viewpoint located above the walking trainee and outputs the image of the walking trainee taken by the first camera 33 to the control device 36 (step S102).

The control device 36 calculates a center line L indicating the center position in the left/right direction of the walking trainee based on the upper-viewpoint image of the walking trainee output from the first camera 33 (step S103).

The control device 36 calculates a target landing point S of the foot of the walking trainee based on the moving speed of the belt 311 of the treadmill 31 and the image of the walking trainee from the upper viewpoint output from the first camera 33 (step 5104).

The control device 36 displays the target landing point S of the foot of the walking trainee and the center line L in the left/right direction of the walking trainee together with the image of the walking trainee from the upper viewpoint taken by the first camera 33 on the display screen of the monitor 35 (step S105). The walking trainee, while moving his/her feet according to their target landing points S displayed on the display screen of the monitor 35, checks the left/right inclination of his/her body by looking at the center line L displayed near the target landing point S of the foot in real time and thereby corrects the inclination. In this way, the walking trainee can, for example, correct his/her habit of inclining to the left/right by himself/herself during the walking training.

As described above, in the walking training apparatus 1 according to the first exemplary embodiment, in addition to the target landing point S of the foot, the center line L indicating the center position in the left/right direction of the walking trainee is displayed on the display screen of the monitor 35. Therefore, the walking trainee can easily check the inclination of his/her body in the left/right direction by looking at the center line L displayed near the target landing point S of his/her foot.

### Second exemplary embodiment

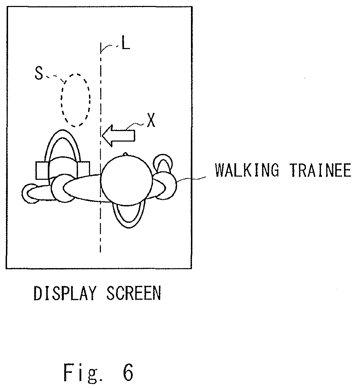

A control device 36 according to a second exemplary embodiment of the present invention calculates the amount of the left/right inclination of the body of the walking trainee and also calculates (i.e., determines) the inclination direction of the body of the walking trainee. Further, the control device 36 displays a mark X that indicates the calculated inclination amount (the amount of the inclination correction) and a counter-inclination direction (the direction of the inclination correction), i.e., a direction opposite to the inclination direction, adjacent to (or next to) the center line L in the left/right direction of the walking trainee on the display screen of the monitor 35 (Fig. 6). Alternatively, the control device 36 may display a mark X that indicates the calculated inclination amount (the amount of the inclination correction) and the inclination direction (the actual inclination direction) adjacent to (or next to) the center line L in the left/right direction of the walking trainee on the display screen of the monitor 35. In this way, the walking trainee can visually recognize the inclination amount of his/her body in the left/right direction and the inclination direction thereof just by looking at the mark X displayed near the target landing point S of his/her foot and the center line L, and thereby check the left/right inclination of his/her body more easily.

The control device 36 is a specific example of the inclination calculation means. The control device 36 can calculate (or determine) the amount of the left/right inclination of the body of the walking trainee and the inclination direction thereof based on information detected by at least one of the pressure sensors 28 of the walking assistance apparatus 2, the gyro-sensor 4, the second camera 34, and the first camera 33.

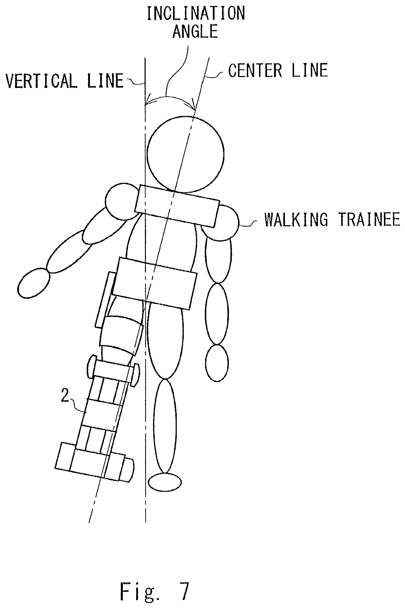

For example, the control device 36 calculates (or determines) the amount of the left/right inclination of the body of the walking trainee and the inclination direction thereof based on the inclination angular speed of the trunk (or torso) of the walking trainee detected by the gyro-sensor 4. Alternatively, the control device 36 calculates the center line in the left/right direction of the trunk based on the image of the walking trainee from the front viewpoint or the rear viewpoint output from the second camera 34 (Fig. 7). Then, the control device 36 calculates the inclination direction and the inclination angle of the calculated center line with respect to the vertical line.

The control device 36 displays, for example, an arrow adjacent to (or next to) the center line in the left/right direction of the walking trainee as the mark X indicating the calculated inclination amount, and the calculated (or determined) inclination direction or the counter-inclination direction on the display screen of the monitor 35 (Fig. 6). The direction of this arrow indicates the inclination direction or the counter-inclination direction and the length of the arrow indicates the magnitude of the inclination amount. Note that the above-described mark is merely an example and the mark is not limited to this example. That is, any arbitrary mark can be applied, provided that the walking trainee can easily recognize the amount of the left/right inclination of the body of the walking trainee and the inclination direction thereof.

Note that by switching the switch 37, the mark X may be disappeared (i.e., vanished) from the display screen of the monitor 35 and only the target landing point S of the foot and the center line L may be displayed on the display screen of the monitor 35. In this way, the walking trainee can do a walking training while, for example, concentrating on instructions given by a physical therapist or the like.

As described above, according to the second exemplary embodiment, a mark X that indicates the amount of the left/right inclination of the body of the walking trainee and the inclination direction thereof is displayed adjacent to (or next to) the center line L in the left/right direction of the walking trainee on the display screen of the monitor 35. In this way, the walking trainee can easily check the left/right inclination of his/her body by looking at the mark X displayed near the target landing point S of his/her foot and the center line L.

### Third exemplary embodiment

A control device 36 according to a third exemplary embodiment of the present invention may notify a walking trainee of at least one of the amount of the left/right inclination of the body of the walking trainee and the inclination direction thereof by a sound produced by a speaker. The speaker is a specific example of the sound output means.

For example, the control device 36 changes the frequency, the volume, the tone, and/or the like of the sound output from the speaker according to the amount of the left/right inclination of the body of the walking trainee and the inclination direction thereof. More specifically, the control device 36 raises the frequency or raises the volume of the sound output from the speaker as the amount of the left/right inclination the body of the walking trainee increases. The control device 36 outputs a sound from a right-side speaker when the body of the walking trainee is inclined to the right and outputs a sound from a left-side speaker when the body of the walking trainee is inclined to the left. Alternatively, the control device 36 may change the tone of the sound output from the speaker according to whether the body of the walking trainee is inclined to the right or to the left.

As described above, according to the third exemplary embodiment, the walking trainee is notified of at least one of the amount of the left/right inclination of his/her body and the inclination direction thereof by using a sound produced by a speaker. In this way, the walking trainee can recognize the inclination amount of his/her body in the left/right direction and the inclination direction thereof more intuitively, and thereby check the left/right inclination of his/her body more easily.

Note that the control device 36 may notify the walking trainee of at least one of the amount of the left/right inclination of the body of the walking trainee and the inclination direction thereof by a vibration produced by a vibration device. The vibration device a specific example of the vibration means.

The control device 36 changes the frequency, the magnitude (i.e., the amplitude), and/or the like of the vibration produced by the vibration device according to the amount of the left/right inclination of the body of the walking trainee and the inclination direction thereof. More specifically, the control device 36 raises the frequency or raises the amplitude of the vibration of the vibration device as the amount of the left/right inclination the body of the walking trainee increases. The control device 36 vibrates the right side of the walking trainee when the body of the walking trainee is inclined to the right and vibrates the left side of the walking trainee when the body of the walking trainee is inclined to the left.

The control device 36 changes the blinking frequency, the brightness, the color, and/or the like of light emitted from a lighting device according to the amount of the left/right inclination of the body of the walking trainee and the inclination direction thereof. The lighting device is a specific example of the optical output means.

More specifically, the control device 36 raises the blinking frequency or raises the brightness of the light emitted from the lighting device as the amount of the left/right inclination the body of the walking trainee increases. The control device 36 turns on a right-side lighting device when the body of the walking trainee is inclined to the right and turns on a left-side lighting device when the body of the walking trainee is inclined to the left. Further, the control device 36 may notify the walking trainee of at least one of the amount of the left/right inclination of the body of the walking trainee and the inclination direction thereof by combining the speaker, the vibration device, and the lighting device in an arbitrary fashion.

Note that the present invention is not limited to the above-described exemplary embodiments, and various modifications can be made.

In the above-described exemplary embodiments, the walking assistance apparatus 2 does not necessarily have to be attached to the leg of the walking trainee.

In the above-described exemplary embodiments, the training apparatus 3 does not necessarily have to be equipped with the frame main body 32. In such a case, the monitor 35 and the first and second cameras 33 and 34 may be disposed (or mounted), for example, on the wall or the ceiling.

From the invention thus described, it will be obvious that the embodiments of the invention may be varied in many ways.

## Claims

1. A walking training apparatus (1) comprising:
display means (35) disposed in front of a walking trainee;
a treadmill (31) which rotates a belt (311) on which the walking trainee walks;
first image pickup means (33) for taking an image of the walking trainee from a viewpoint; and
display control means (36) for calculating a target landing point (S) of the foot of the walking trainee based on a moving speed of the belt (311) and based on the image of the walking trainee, and for controlling the display means (35) so that the target landing point (S) is displayed on a display screen of the display means (35), the target landing point (S) being a point on which the walking trainee should land his/her foot in a next step, **characterized in that**
the first image pickup means (33) is located above the walking trainee;
the image of the walking trainee taken by the first image pickup means (33) is also displayed on the display screen of the display means (35);
the display control means (36) is adapted to control the display means (35) so that a center line (L) in addition to the target landing point (S) of the foot are displayed on the display screen of the display means (35), the center line (L) indicating a center position in a left/right direction of the walking trainee; and
the display control means (36) is adapted to determine the positions of the left and right feet of the walking trainee based on the image of the walking trainee from the upper-viewpoint image output from the first image pickup means (33) and is adapted to calculate a line extending from the center of the positions of the left and right feet in the front/rear direction as the center line (L).

2. The walking training apparatus (1) according to Claim 1, further comprising inclination calculation means (36) for calculating an amount of a left/right inclination and a direction thereof of the body of the walking trainee based on information detected by at least one of a pressure sensor that detects a pressure on a sole of the foot of the walking trainee, an angular speed sensor attached to the walking trainee, second image pickup means (34) for taking an image of the walking trainee from front of or behind the walking trainee, and the first image pickup means (33), wherein
the display control means (36) is adapted to control the display means (35) so that a mark (X) indicating the amount of the inclination calculated by the inclination calculation means (36) and the direction of the inclination or a counter-inclination direction adjacent to the center line (L) are displayed on the display screen of the display means (35).

3. The walking training apparatus (1) according to Claim 2, further comprising at least one of sound output means for changing a sound output according to the inclination amount and the inclination direction calculated by the inclination calculation means (36), vibration means for changing a vibration given to the walking trainee, and optical output means for changing an optical output.

4. The walking training apparatus (1) according to Claim 2, further comprising a walking assistance apparatus (2) having the pressure sensor (28) on the sole thereof.

5. The walking training apparatus (1) according to one of the Claims 1 to 4, further comprising an angular speed sensor (4) attached to the walking trainee, wherein the walking training apparatus (1) is a walking training apparatus for patients having hemiplegia.

6. The walking training apparatus (1) according to one of the Claims 1 to 5, further comprising a switch (37) for switching on and off the center line (L).

7. The walking training apparatus (1) according to Claim 6, wherein said switch (37) is further adapted to switch on and off the mark (X) indicating the calculated amount of the inclination.

8. A walking training method in which a target landing point (S) is displayed on a display screen of display means (35) disposed in front of a walking trainee, an image of the walking trainee being taken by a first image pickup means (33) for taking the image of the walking trainee, the target landing point (S) being a point on which the walking trainee should land his/her foot in a next step, wherein the target landing point (S) is calculated based on a moving speed of the belt (311) and based on the image of the walking trainee,
**characterized in that**
the first image pickup means (33) is located above the walking trainee for taking the image of the walking trainee from a viewpoint located above the walking trainee;
the image of the walking trainee taken by the first image pickup means (33) is displayed also on the display screen of the display means (35); and
in addition to the target landing point (S) of the foot, a center line (L) is displayed on the display screen of the display means (35), the center line (L) indicating a center position in a left/right direction of the walking trainee based on the upper-viewpoint image of the walking trainee output from the first image pickup means (33), the center line (L) being calculated as a line extending from the center of the positions of the left and right feet in the walking direction of the walking trainee.

## Patentansprüche

1. Lauftrainingvorrichtung (1) mit:
einer Anzeigeeinrichtung (35), die vor einem das Laufen Trainierenden angeordnet ist;
einer Laufbandeinrichtung (31), die ein Band (311) dreht, auf dem der das Laufen Trainierende läuft;
einer ersten Bildaufnahmeeinrichtung (33) zum Aufnehmen eines Bildes des das Laufen Trainierenden von einem Betrachtungspunkt; und
einer Anzeigesteuereinrichtung (36) zum Berechnen eines Solllandepunktes (S) des Fußes des das Laufen Trainierenden auf der Basis einer Bewegungsgeschwindigkeit des Bandes (311) und auf der Basis des Bildes des das Laufen Trainierenden, und zum Steuern der Anzeigeeinrichtung (35) derart, dass der Solllandepunkt (S) auf einem Bildschirm der Anzeigeeinrichtung (35) angezeigt wird, wobei der Solllandepunkt (S) ein Punkt ist, auf dem der das Laufen Trainierende seinen Fuß bei einem nächsten Schritt landen lassen soll,
**dadurch gekennzeichnet, dass**
die erste Bildaufnahmeeinrichtung (33) oberhalb des das Laufen Trainierenden angeordnet ist;
das Bild des das Laufen Trainierenden, das durch die erste Bildaufnahmeeinrichtung (33) aufgenommen wird, ebenfalls auf dem Bildschirm der Anzeigeeinrichtung (35) angezeigt wird;
die Anzeigesteuereinrichtung (36) daran angepasst ist, dass sie die Anzeigeeinrichtung (35) so steuert, dass eine Mittellinie (L) zusätzlich zu dem Solllandepunkt (S) des Fußes auf dem Bildschirm der Anzeigeeinrichtung (35) angezeigt wird, wobei die Mittellinie (L) eine Mittenposition in einer nach links und nach rechts weisenden Richtung des das Laufen Trainierende anzeigt; und
die Anzeigesteuereinrichtung (36) daran angepasst ist, dass sie die Positionen des linken Fußes und des rechten Fußes des das Laufen Trainierenden auf der Basis des Bildes des das Laufen Trainierenden aus dem Bild des oberen Betrachtungspunktes bestimmt, das von der ersten Bildaufnahmeeinrichtung (33) ausgegeben wird, und daran angepasst ist, eine Linie, die sich von der Mitte der Positionen des linken und rechten Fußes in der nach vorn und nach hinten weisenden Richtung erstreckt, als die Mittellinie (L) zu berechnen.

2. Lauftrainingvorrichtung (1) gemäß Anspruch 1, die des Weiteren eine Neigungsberechnungseinrichtung (36) aufweist für ein Berechnen eines Betrages einer nach links/rechts erfolgenden Neigung und seiner Richtung des Körpers des das Laufen Trainierenden auf der Basis einer Information, die durch zumindest entweder einem Drucksensor, der einen Druck an einer Sohle des Fußes des das Laufen Trainierenden erfasst, einem Winkelgeschwindigkeitssensor, der am das Laufen Trainierenden angebracht ist, einer zweiten Bildaufnahmeeinrichtung (34) zum Aufnehmen eines Bildes des das Laufen Trainierenden von der Vorderseite oder der Rückseite des das Laufen Trainierenden und/oder der ersten Bildaufnahmeeinrichtung (33) erfasst wird, wobei
die Anzeigesteuereinrichtung (36) daran angepasst ist, die Anzeigeeinrichtung (35) so zu steuern, dass eine Markierung (X), die den Betrag der Neigung anzeigt, die durch die Neigungsberechnungseinrichtung (36) berechnet wird, und die Richtung der Neigung oder eine Gegenneigungsrichtung benachbart zu der Mittellinie (L), auf dem Bildschirm der Anzeigeeinrichtung (35) dargestellt werden.

3. Lauftrainingvorrichtung (1) gemäß Anspruch 2, die des Weiteren zumindest entweder eine Tonausgabeeinrichtung zum Ändern eines Tons, der gemäß dem Neigungsbetrag und der Neigungsrichtung ausgegeben wird, die durch die Neigungsberechnungseinrichtung (36) berechnet werden, eine Schwingungseinrichtung zum Ändern einer Schwingung, die dem das Laufen Trainierenden mitgeteilt wird, und/oder eine optische Ausgabeeinrichtung zum Ändern einer optischen Ausgabe aufweist.

4. Lauftrainingvorrichtung (1) gemäß Anspruch 2, die des Weiteren eine Laufunterstützungsvorrichtung (2) aufweist, die den Drucksensor (28) an ihrer Sohle aufweist.

5. Lauftrainingvorrichtung (1) gemäß einem der Ansprüche 1 bis 4, die des Weiteren einen Winkelgeschwindigkeitssensor (4) aufweist, der an dem das Laufen Trainierenden angebracht ist, wobei die Lauftrainingvorrichtung (1) eine Lauftrainingvorrichtung für Patienten mit Hemiplegie ist.

6. Lauftrainingvorrichtung (1) gemäß einem der Ansprüche 1 bis 5, die des Weiteren einen Schalter (37) zum Einschalten und Ausschalten der Mittellinie (L) aufweist.

7. Lauftrainingvorrichtung (1) gemäß Anspruch 6, wobei der Schalter (37) des Weiteren daran angepasst ist, die Markierung (X) einzuschalten und auszuschalten, die den berechneten Betrag der Neigung anzeigt.

8. Lauftrainingverfahren, bei dem ein Solllandepunkt (S) auf einem Bildschirm einer Anzeigeeinrichtung (35) angezeigt wird, die vor einem ein Laufen Trainierenden angeordnet ist, wobei ein Bild des das Laufen Trainierenden durch eine erste Bildaufnahmeeinrichtung (33) zum Aufnehmen des Bildes des das Laufen Trainierenden aufgenommen wird, wobei der Solllandepunkt (S) ein Punkt ist, auf dem der das Laufen Trainierende seinen Fuß bei einem nächsten Schritt landen lassen soll, wobei der Solllandepunkt (S) auf der Basis einer Bewegungsgeschwindigkeit des Bandes (311) und auf der Basis des Bildes des das Laufen Trainierenden berechnet wird,
**dadurch gekennzeichnet, dass**
die erste Bildaufnahmeeinrichtung (33) oberhalb des das Laufen Trainierenden angeordnet ist für ein Aufnehmen des Bildes des das Laufen Trainierenden von einem Betrachtungspunkt, der sich über dem des Laufen Trainierenden befindet;
das Bild des das Laufen Trainierenden, das durch die erste Bildaufnahmeeinrichtung (33) aufgenommen wird, ebenfalls auf dem Bildschirm der Anzeigeeinrichtung (35) dargestellt wird; und
zusätzlich zu dem Solllandepunkt (S) des Fußes eine Mittellinie (L) auf dem Bildschirm der Anzeigeeinrichtung (35) dargestellt wird, wobei die Mittellinie (L) eine Mittenposition in einer nach links und nach rechts weisenden Richtung des das Laufen Trainierenden auf der Basis des Bildes des von oben bewirkten Betrachtungspunktes des das Laufen Trainierenden anzeigt, das von der ersten Bildaufnahmeeinrichtung (33) ausgegeben wird, wobei die Mittellinie (L) als eine Linie berechnet wird, die sich von der Mitte der Positionen des linken und rechten Fußes in der Laufrichtung des das Laufen Trainierenden erstreckt.

## Revendications

1. Appareil d'entraînement à la marche (1) comprenant :
un moyen d'affichage (35) disposé en face d'une personne qui s'entraîne à la marche ;
un tapis roulant (31) qui fait tourner une courroie (311) sur laquelle marche la personne qui s'entraîne à la marche ;
un premier moyen de prise d'image (33) pour prendre une image de la personne qui s'entraîne à la marche d'un point de vue ; et
un moyen de commande d'affichage (36) pour calculer un point d'appui cible (S) du pied de la personne qui s'entraîne à la marche sur la base d'une vitesse de déplacement de la courroie (311) et sur la base de l'image de la personne qui s'entraîne à la marche, et pour contrôler le moyen d'affichage (35) de sorte que le point d'appui cible (S) est affiché sur un écran d'affichage du moyen d'affichage (35), le point d'appui cible (S) étant un point sur lequel la personne qui s'entraîne à la marche doit poser son pied au pas suivant, **caractérisé en ce que** :
le premier moyen de prise d'image (33) est positionné au-dessus de la personne qui s'entraîne à la marche ;
l'image de la personne qui s'entraîne à la marche prise par le premier moyen de prise d'image (33) est également affichée sur l'écran d'affichage du moyen d'affichage (35) ;
le moyen de contrôle d'affichage (36) est adapté pour contrôler le moyen d'affichage (35) de sorte qu'une ligne centrale (L) en plus du point d'appui cible (S) du pied, sont affichés sur l'écran d'affichage du moyen d'affichage (35), la ligne centrale (L) indiquant une position centrale dans une direction gauche / droite de la personne qui s'entraîne à la marche ; et
le moyen de contrôle d'affichage (36) est adapté pour déterminer les positions des pieds gauche et droit de la personne qui s'entraîne à la marche sur la base de l'image de la personne qui s'entraîne à la marche d'après l'image du point de vue supérieur produite par le premier moyen de prise d'image (33) et est adapté pour calculer une ligne s'étendant à partir du centre des positions des pieds gauche et droit dans la direction avant / arrière, en tant que ligne centrale (L).

2. Appareil d'entraînement à la marche (1) selon la revendication 1, comprenant en outre un moyen de calcul d'inclinaison (36) pour calculer une quantité d'inclinaison gauche / droite et la direction du corps de la personne qui s'entraîne à la marche sur la base de l'information détectée par au moins l'un parmi un capteur de pression qui détecte une pression sur la plante du pied de la personne qui s'entraîne à la marche, un capteur de vitesse angulaire fixé sur la personne qui s'entraîne à la marche, un second moyen de prise d'image (34) pour prendre une image de la personne qui s'entraîne à la marche depuis le devant ou le derrière de la personne qui s'entraîne à la marche, et le premier moyen de prise d'image (33), dans lequel :
le moyen de contrôle d'affichage (36) est adapté pour contrôler le moyen d'affichage (35) de sorte qu'une marque (X) indiquant la quantité de l'inclinaison calculée par le moyen de calcul d'inclinaison (36) et la direction de l'inclinaison ou la direction d'une inclinaison antagoniste adjacente à la ligne centrale (L) sont affichées sur l'écran d'affichage du moyen d'affichage (35).

3. Appareil d'entraînement à la marche (1) selon la revendication 2, comprenant en outre au moins un moyen de sortie de son pour modifier une sortie de son selon la quantité d'inclinaison et la direction d'inclinaison calculées par le moyen de calcul d'inclinaison (36), un moyen de vibration pour modifier une vibration transmise à la personne qui s'entraîne à la marche, et un moyen de sortie optique pour modifier une sortie optique.

4. Appareil d'entraînement à la marche (1) selon la revendication 2, comprenant en outre un appareil d'assistance à la marche (2) ayant un capteur de pression (28) sur sa plante de pied.

5. Appareil d'entraînement à la marche (1) selon l'une des revendications 1 à 4, comprenant en outre un capteur de vitesse angulaire (4) fixé sur la personne qui s'entraîne à la marche, dans lequel l'appareil d'entraînement à la marche (1) est un appareil d'entraînement à la marche pour des patients atteints d'hémiplégie.

6. Appareil d'entraînement à la marche (1) selon l'une des revendications 1 à 5, comprenant en outre un commutateur (37) pour activer et désactiver la ligne centrale (L).

7. Appareil d'entraînement à la marche (1) selon la revendication 6, dans lequel ledit commutateur (37) est en outre adapté pour activer et désactiver la marque (X) indiquant la quantité calculée d'inclinaison.

8. Procédé d'entraînement à la marche dans lequel un point d'appui cible (S) est affiché sur un écran d'affichage du moyen d'affichage (35) disposé en face d'une personne qui s'entraîne à la marche, une image de la personne qui s'entraîne à la marche étant prise par un premier moyen de prise d'image (33) pour prendre l'image de la personne qui s'entraîne à la marche, le point d'appui cible (S) étant un point sur lequel la personne qui s'entraîne à la marche pose son pied lorsque du pas suivant, dans lequel le point d'appui cible (S) est calculé sur la base d'une vitesse de déplacement de la courroie (311) et sur la base de l'image de la personne qui s'entraîne à la marche,
**caractérisé en ce que** :
le premier moyen de prise d'image (33) est positionné au-dessus de la personne qui s'entraîne à la marche pour prendre l'image de la personne qui s'entraîne à la marche d'un point de vue positionné au-dessus de la personne qui s'entraîne à la marche ;
l'image de la personne qui s'entraîne à la marche prise par le premier moyen de prise d'image (33) est affichée uniquement sur l'écran d'affichage du moyen d'affichage (35) ; et
en plus du point d'appui cible (S) du pied, une ligne centrale (L) est affichée sur l'écran d'affichage du moyen d'affichage (35), la ligne centrale (L) indiquant une position centrale dans une direction gauche / droite de la personne qui s'entraîne à la marche sur la base de l'image du point de vue supérieur de la personne qui s'entraîne à la marche produite par le premier moyen de prise d'image (33), la ligne centrale (L) étant calculée comme étant une ligne s'étendant à partir du centre des positions des pieds gauche et droit dans la direction de marche de la personne qui s'entraîne à la marche.
